# EUROPEAN PATENT SPECIFICATION

(11) **EP 3 764 439 B1**
(45) Date of publication and mention of the grant of the patent: **20.09.2023**
(21) Application number: 19846615.3
(22) Date of filing: 29.07.2019
(51) Int. Cl.: H01M 4/36, H01M 4/62, H01M 4/38, H01M 4/58, H01M 4/60, H01M 10/052, H01M 10/42, H01M 4/02

(54) **SULFUR-CARBON COMPOSITE, PREPARATION METHOD THEREFOR, AND POSITIVE ELECTRODE AND LITHIUM SECONDARY BATTERY COMPRISING SAME**
SCHWEFEL-KOHLENSTOFF-VERBUNDWERKSTOFF, VERFAHREN ZU SEINER HERSTELLUNG SOWIE POSITIVE ELEKTRODE UND LITHIUM-SEKUNDÄRBATTERIE DAMIT
COMPOSITE SOUFRE-CARBONE, SON PROCÉDÉ DE PRÉPARATION, ET ÉLECTRODE POSITIVE ET ACCUMULATEUR AU LITHIUM LE COMPRENANT

(30) Priority: 08.08.2018 KR 20180092206; 26.07.2019 KR 20190090686
(43) Date of publication of application: 13.01.2021
(73) Proprietor: LG Energy Solution, Ltd., Seoul 07335 (KR)
(72) Inventor: KIM, Bong Soo, Daejeon 34122 (KR); KIM, Soohyun, Daejeon 34122 (KR); JIN, Sun Mi, Daejeon 34122 (KR)
(74) Representative: Hoffmann Eitle
(86) International application number: PCT/KR2019/009380
(87) International publication number: WO 2020/032454

(56) References cited:
- WO-A1-2008/099873
- WO-A1-2015/059155
- WO-A2-2018/084449
- KR-A- 20070 088 989
- KR-A- 20140 001 935
- KR-A- 20150 061 874
- KR-A- 20150 061 874
- KR-A- 20160 075 590
- US-A1- 2004 009 396
- LIU ZHIXUAN ET AL: "Hierarchical nitrogen-doped porous graphene/reduced fluorographene/sulfur hybrids for high-performance lithium-sulfur batteries", PHYSICAL CHEMISTRY CHEMICAL PHYSICS, [Online] vol. 19, no. 3, 19 December 2016 (2016-12-19), pages 2567-2573, XP055801675, ISSN: 1463-9076, DOI: 10.1039/C6CP07650E Retrieved from the Internet: URL:https://pubs.rsc.org/en/content/articl epdf/2017/cp/c6cp07650e> [retrieved on 2021-05-05]

## Description

### [Technical Field]

The present invention relates to a sulfur-carbon composite, a method for preparing the same, and a positive electrode and lithium secondary battery comprising the same.

### [Background Art]

Recently, with the rapid development of the field of electronic devices and electric vehicles, the demand for secondary batteries is increasing. In particular, with the trend of miniaturization and light weight of portable electronic devices, the demand for secondary batteries having a high energy density capable of responding thereto is increasing.

Among the secondary batteries, the lithium-sulfur secondary battery is a secondary battery which uses a sulfur-based compound having a sulfur-sulfur bond as a positive electrode active material and uses alkali metals such as lithium, or carbon-based materials where intercalation and deintercalation of metal ions such as lithium ions occur, or silicon or tin forming alloy with lithium, as a negative electrode active material. Specifically, the lithium-sulfur secondary battery stores and generates electrical energy by using the oxidation-reduction reaction in which during the discharging which is a reduction reaction, the oxidation number of sulfur is reduced while sulfur-sulfur bonds are broken, and during the charging which is an oxidation reaction, the sulfur-sulfur bond is formed again while the oxidation number of sulfur is increased.

In particular, since sulfur used as a positive electrode active material in lithium-sulfur secondary battery has a theoretical energy density of 1,675 mAh/g, which is 5 times higher than the positive electrode active material used in the conventional lithium secondary battery, the lithium-sulfur secondary battery is a battery capable of expressing high power and high energy density. In addition, since sulfur has the advantages of low cost, rich reserves, easy supply, and environmental friendliness, sulfur is attracting attention as an energy source for medium and large devices such as electric vehicles as well as portable electronic devices.

However, since sulfur has an electrical conductivity of 5×10⁻³⁰ S/cm, which is a nonconductor without electrical conductivity, there is a problem that the movement of electrons generated by the electrochemical reaction is difficult. Accordingly, sulfur is compounded with an electrically conductive material such as carbon that can provide an electrochemical reaction site, and the sulfur-carbon composite produced thereby is used.

In the existing sulfur-carbon composites, there was a problem that sulfur is leaked into the electrolyte during the oxidation-reduction reaction, and thus the lifetime of the battery is deteriorated, and also lithium polysulfide which is a reduced substance of sulfur is leached, causing sulfur to no longer participate in the electrochemical reaction. In addition, there is a problem that when sulfur is loaded into the electrode in excess, the capacity is reduced.

As a means to solve these problems, research and development are continuously attempted to diversify the positive electrode material of the lithium-sulfur secondary battery.

As an example, Japanese Patent Publication No. 2016-534496 describes a composition for forming an electrode for a lithium-sulfur secondary battery, which comprises a powder type electrode forming material composed of sulfur, an electrically conductive material based on carbon, and at least one surfactant selected from fluorinated surfactants and the like, and discloses that due to the positive electrode comprising such a composition, the lithium-sulfur secondary battery can exhibit excellent capacity values during charging and discharging cycles of the battery.

In addition, US Patent Publication No. 2014-0079989 discloses that sulfur, carbon black, graphite, and the like, and an electrically conductive additive can be contained in the positive electrode of a lithium-sulfur secondary battery, and a fluorine-based surfactant can be further contained to improve the lifetime of the electrode.

However, in the case of the lithium-sulfur secondary battery disclosed in the above-mentioned documents, there is still no solution that can prevent the lowering phenomenon of the reaction rate due to the viscosity which increases when the polysulfide is dissolved in electrolyte solution. Therefore, there is an urgent need to develop a positive electrode material that can improve this.

WO 2018/084449 A2 describes a composite comprising: a porous carbon material; and sulfur at at least a part of the surface and the inside of the porous carbon material, wherein the porous carbon material includes ion conductive polymer-containing coating layers on the inner and the outer surface thereof.

KR 2015 0061874 A describes a positive electrode for a lithium-sulfur battery that comprises: a conductor comprising a carbon nanotube aggregate; and a positive electrode active material. The document states that a role to trap polysulfide may be enhanced by coating a carbon nanotube with an amphipathic material having hydrophilic properties or both of hydrophilic and hydrophobic properties.

WO 2015/059155 A1 describes an electrode-forming composition comprising an aqueous latex comprising at least one fluoropolymer comprising recurring units derived from vinylidene fluoride, at least one hydrogenated monomer and, optionally, at least one other fluorinated monomer [different from VDF, and, homogeneously dispersed therein, (b) at least one powdery electrode-forming material consisting of Sulphur. The composition further comprises at least one powdery electrically conducting material, optionally at least one surfactant, optionally at least one binding agent and optionally less than 10% by weight of at least one organic solvent.

### [Prior Art Document]

(Patent Document 1) Japanese Patent Publication No. 2016-534496
(Patent Document 2) US Patent Publication No. 2014-0079989

### [Disclosure]

### [Technical Problem]

As a result of various studies to solve the above problems, the inventors of the present invention have developed a sulfur-carbon composite containing fluorine-based surfactant (fluorosurfactant) which is a material that can lower the surface energy of organic solvents contained in electrolyte solution, wherein the sulfur-carbon composite is in a form in which the fluorine-based surfactant and sulfur each form a double coating layer on the surface of carbon. When applying the positive electrode containing such a sulfur-carbon composite to a lithium secondary battery, the life of the battery may be increased, and the lowering phenomenon of the reaction rate by the increase in the viscosity due to the polysulfide dissolved in an electrolyte solution may be alleviated.

Therefore, it is an object of the present invention to provide a sulfur-carbon composite which can prevent deterioration of lithium and lower the surface energy of an electrolyte solution, and a method of manufacturing the same.

It is another object of the present invention to provide a positive electrode comprising the sulfur-carbon composite as described above.

It is still another object of the present invention to provide a lithium secondary battery comprising the positive electrode which comprises the sulfur-carbon composite as described above.

### [Technical Solution]

In order to achieve the above objects, the present invention provides a sulfur-carbon composite comprising a porous carbon material, a fluorine-based surfactant coating layer formed on a surface of the porous carbon material, and a sulfur coating layer formed on a surface of the fluorine-based surfactant coating layer opposite the porous carbon material.

The sulfur-carbon composite comprises 9 wt.% to 49 wt.% of the porous carbon material, 0.1 wt.% to 3 wt.% of the fluorine-based surfactant, and 50 wt.% to 90 wt.% of sulfur.

The porous carbon material may comprise one or more selected from the group consisting of graphite, graphene, carbon black, carbon nanotubes, carbon fibers, and activated carbon.

The fluorine-based surfactant is represented by Formula 1 below: 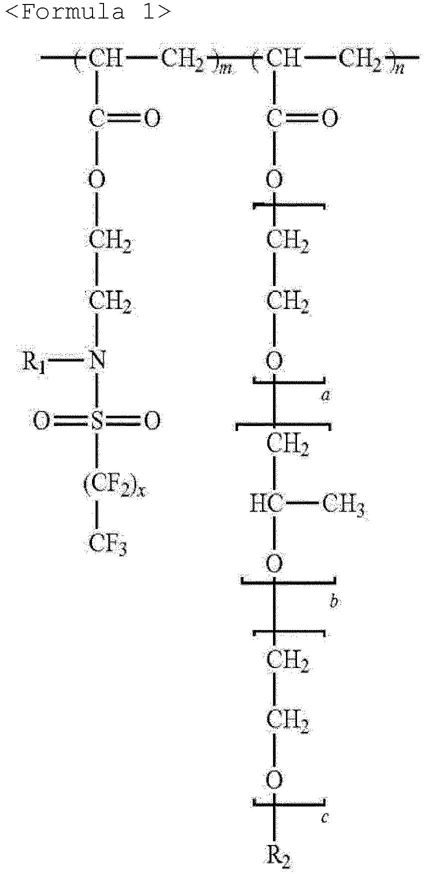 wherein m/(m + n) is a real number from 0 to 1 (wherein m and n are each an integer); R₁ is hydrogen or an alkyl group having 1 to 18 carbon atoms; R₂ is the connection point for hydrogen, an alkyl group having 1 to 18 carbon atoms, or an acrylate polymer backbone; x is an integer from 1 to 10; a is an integer from 1 to 50; b is an integer from 1 to 100; and c is an integer from 1 to 50.

The sulfur may be at least one selected from the group consisting of inorganic sulfur (S₈), Li₂Sₙ(n ≥ 1, n is an integer), organic sulfur compound, and carbon-sulfur polymer [(C₂Sₓ)ₙ, 2.5 ≤ x ≤ 50, n ≥ 2, x and n are integers].

The present invention also provides a method for preparing a sulfur-carbon composite comprising the steps of (S1) dissolving the fluorine-based surfactant (fluorosurfactant) represented by Formula 1 in a solvent to prepare a fluorine-based surfactant solution; (S2) preparing a slurry by mixing the fluorine-based surfactant solution with a porous carbon material; (S3) drying the slurry to remove the solvent and form a slurry powder; (S4) mixing the slurry powder and sulfur to form a mixture; and (S5) heating the mixture at a heating temperature of 115 °C or more to produce a sulfur-carbon composite.

In step (S1), the solvent may be at least one selected from the group consisting of methanol, ethanol, 1-propanol, 2-butanol, isobutanol, 3-pentanol, dodecanol, chloroform, carbon tetrachloride, trichloroethylene, tetrachloroethylene, N-methyl-2-pyrrolidinone (NMP), and dimethyl formamide (DMF).

The concentration of the fluorine-based surfactant solution may be 0.1 wt.% to 10 wt.% based on a tital weight of solids.

In step (S3), the drying may be performed by stirring the slurry or heating the slurry while stirring under vacuum.

In step (S5), the heating temperature is at least 115 °C.

The present invention also provides a positive electrode for a lithium secondary battery comprising the sulfur-carbon composite.

The present invention also provides a lithium secondary battery comprising the positive electrode, and the lithium secondary battery may be a lithium-sulfur secondary battery.

### [Advantageous Effects]

According to the sulfur-carbon composite of the present invention, the sulfur-carbon composite may contain an appropriate amount of the fluorine-based surfactant, thereby preventing deterioration of lithium by the fluorine-based surfactant. In addition, the sulfur-carbon composite improves the surface wetting properties by lowering the surface energy of the organic solvent contained in the electrolyte solution, and thus even if the viscosity of the electrolyte solution is increased due to the polysulfide leaching from the positive electrode, the reduction of the reaction rate in the positive electrode and the negative electrode can be alleviated, thereby inducing a uniform reaction in the positive electrode and the negative electrode, and improving the lifetime of the lithium secondary battery.

In addition, according to the present invention, by introducing the fluorine-based surfactant to the sulfur-carbon composite which is a positive electrode material of a lithium secondary battery, particularly, a lithium-sulfur secondary battery, a more uniform sulfur distribution can be induced in the complexing process of melting sulfur at high temperatures.

In addition, according to the present invention, by introducing the fluorine-based surfactant to the sulfur-carbon composite which is a positive electrode material of a lithium secondary battery, particularly, a lithium-sulfur secondary battery, it is possible to prevent the reduction of the discharging capacity by allowing the small amount to diffuse slowly in the discharging process of the battery.

### [Description of Drawings]

FIGs. 1a and 1b are schematic views showing cross sections of a sulfur-carbon composite according to one embodiment of the present invention.
FIG. 2 is a graph showing the discharging capacity measured for the lithium-sulfur secondary batteries prepared in Example 1 of the present invention and Comparative Example 1.
FIG. 3 is a graph showing the lifetime characteristics measured for the lithium-sulfur secondary batteries prepared in Example 1 of the present invention and Comparative Example 1.
FIG. 4 is a graph showing the discharging capacity measured for the lithium-sulfur secondary batteries prepared in Comparative Example 1 and Comparative Example 2 of the present disclosure.
FIG. 5 is a graph showing the discharging capacity measured for the lithium-sulfur secondary batteries prepared in Comparative Example 1 and Comparative Example 3 of the present disclosure.
FIG. 6 is a graph showing the lifetime characteristics measured for the lithium-sulfur secondary batteries prepared in Example 2 and Comparative Example 1 of the present disclosure.
FIG. 7 is a graph showing the discharging capacity measured for the lithium-sulfur secondary batteries prepared in Example 3 of the present disclosure and Comparative

### Example 1.

FIGs. 8a and 8b are Scanning Electron Microscope (SEM) photographs of the cross section of the sulfur-carbon composite prepared in Example 1.

### [Best Mode]

Hereinafter, the present invention will be described in more detail to assist understanding of the present invention.

The terms and words used in the present specification and claims should not be construed as limited to ordinary or dictionary terms, and should be construed in a sense and concept consistent with the technical idea of the present invention, based on the principle that the inventor can properly define the concept of a term to describe his invention in the best way possible.

The term "composite" as used herein refers to a material that two or more materials are combined to express a more effective function while forming physically and chemically different phases to each other.

### Sulfur-carbon composite

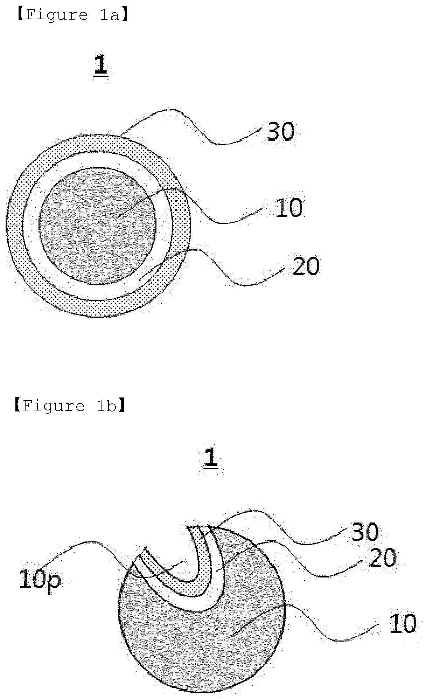

FIGs. 1a and 1b show cross sections of a sulfur-carbon composite according to one embodiment of the present invention.

Referring to FIG. 1a, the sulfur-carbon composite 1 according to the present invention may comprise a porous carbon material 10; a fluorine-based surfactant coating layer 20 formed on the surface of the porous carbon material 10; and a sulfur coating layer 30 formed on the surface of the fluorine-based surfactant coating layer 20.

Also, referring to FIG. 1b, the sulfur-carbon composite 1 according to the present invention may comprise a fluorine-based surfactant coating layer 20 formed on the inner surface of the pores 10p of the porous carbon material 10; and a sulfur coating layer 30 formed on the surface of the fluorine-based surfactant coating layer 20.

In the present invention, the porous carbon material can be generally produced by carbonizing precursors of various carbon materials.

The porous carbon material may comprise uneven pores therein, the average diameter of the pores is in the range of 1 to 200 nm, preferably 1 to 150 nm, more preferably 1 to 100 nm, and the porosity may be in the range of 10 to 90%, preferably 10 to 80%, more preferably 10 to 70% of the total volume of the porosity. If the average diameter of the pores and the porosity are less than the above ranges, the pore size is only at the molecular level, and impregnation with sulfur is impossible. On the contrary, if the average diameter of the pores and the porosity exceed the above ranges, the mechanical strength of the porous carbon is weakened, which is not preferable for application to the manufacturing process of the electrode.

The shape of the porous carbon material is in the form of sphere, rod, needle, plate, tube, or bulk, and can be used without limitation as long as it is commonly used in a lithium secondary battery.

The porous carbon material may have a porous structure or a high specific surface area, and may be any of those conventionally used in the art. For example, the porous carbon material may be, but is not limited to, at least one selected from the group consisting of graphite; graphene; carbon blacks such as Denka black, acetylene black, Ketjen black, channel black, furnace black, lamp black, and thermal black; carbon nanotubes (CNTs) such as single wall carbon nanotube (SWCNT), and multiwall carbon nanotubes (MWCNT); carbon fibers such as graphite nanofiber (GNF), carbon nanofiber (CNF), and activated carbon fiber (ACF); and activated carbon. Preferably, the porous carbon material may be graphite.

In addition, the porous carbon material is contained in an amount of 9 to 49 wt.%, and may preferably be contained in an amount of 15 to 40 wt.%, more preferably 20 to 30 wt.% based on the total weight of the sulfur-carbon composite. If the amount of the porous carbon material is less than the above range, the surface area and space for impregnating sulfur is insufficient, thereby degrading the electrochemical utilization (reactivity) of sulfur. If the amount of the porous carbon material exceeds the above range, when applied to the positive electrode of the lithium secondary battery, since the content of fluorine-based surfactant and sulfur contained in the sulfur-carbon composite is relatively decreased, the energy density of the battery may be excessively reduced.

The fluorine-based surfactant is represented by Formula 1 below. 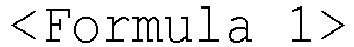 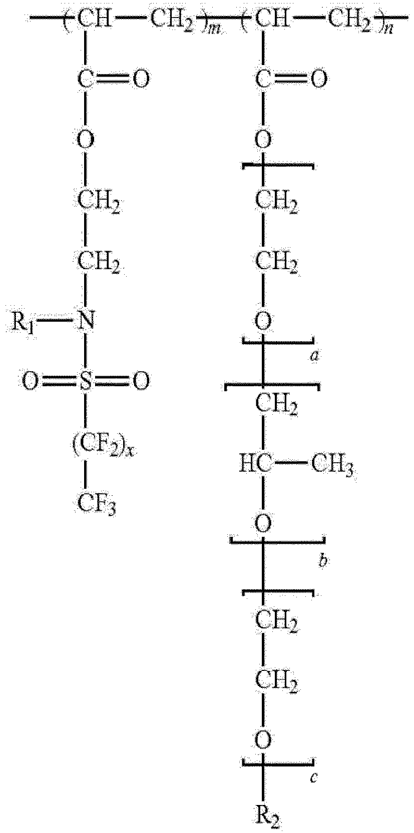 wherein m/ (m+n) is 0 to 1; R₁ is hydrogen or an alkyl group having 1 to 18 carbon atoms; R₂ is the connection point for hydrogen, an alkyl group having 1 to 18 carbon atoms, or an acrylate polymer backbone; x is an integer from 1 to 10; a is an integer from 1 to 50; b is an integer from 1 to 100; and c is an integer from 1 to 50.

In addition, the fluorine-based surfactant is contained in an amount of 0.1 to 3 wt.%, and may preferably be contained in an amount of 0.1 to 2 wt.%, more preferably 0.1 to 1 wt.% based on the total weight of the sulfur-carbon composite. If the amount of the fluorine-based surfactant is less than the above range, the fluorine-based surfactant coating is not sufficient to coat the surface of the carbon material, and the effect of lowering the surface energy of the electrolyte solution is insignificant, and thus the effect of improving the lifetime characteristics of the battery is not significant. If the amount of the fluorine-based surfactant is greater than the above range, the surplus surfactant may act as an electrical resistance, and the contents of sulfur and carbon in the sulfur-carbon composite may be relatively decreased, thereby degrading the performance of the battery.

In addition, the fluorine-based surfactant may be formed in the form of a coating layer on the porous carbon material. Since the fluorine-based surfactant itself has a property of lowering the surface energy of the organic solvent contained in the electrolyte solution, even if the viscosity of the electrolyte solution is increased by the polysulfide leached from the sulfur coating layer formed on the surface of the fluorine-based surfactant coating layer, the wettability of the electrolyte solution to the positive electrode can be improved by the fluorine-based surfactant coating layer. In addition, by acting as an interfacial stabilizer to form a molecular film on the surface of the negative electrode in the charging/discharging process, it is possible to improve the lifetime by making the shape of lithium metal constant and thus delaying deterioration. The fluorine-based surfactant coating layer may prevent the lithium contained in the negative electrode from being deteriorated by the polysulfide eluted from the sulfur coating layer.

In the present invention, the sulfur may be at least one selected from the group consisting of inorganic sulfur (S₈), Li₂Sₙ (n ≥ 1, n is an integer), organic sulfur compound, and carbon-sulfur polymer [(C₂Sₓ)ₙ, 2.5 ≤ x ≤ 50, n ≥ 2, x and n are integers].

The sulfur is contained in an amount of 50 to 90 wt.%, and may preferably be contained in an amount of 60 to 85 wt.%, more preferably 70 to 85 wt.%, based on the total weight of the sulfur-carbon composite. If the amount is less than the above range, the percentage of sulfur which is an electrochemical active material may be reduced, and thus the capacity of the battery may be too small. In addition, if the amount exceeds the above range, the non-conductive sulfur blocks the conductive structure of the carbon material, thereby blocking the electrochemical activity, and thus limiting the operation of the battery.

In addition, the sulfur coating layer may be formed on the surface of the fluorine-based surfactant coating layer in the form of islands by the aggregation of sulfur, and may be formed in a uniform layer form to cover all of the fluorine-based surfactant coating layer. Preferably, when applied to a positive electrode of a lithium secondary battery, for example, a lithium-sulfur secondary battery, the sulfur coating layer may be formed in a uniform layer form in consideration of the capacity of the battery.

The sulfur-carbon composite as described above may be prepared by sequentially forming a fluorine-based surfactant coating layer and a sulfur coating layer on a porous carbon material, and may be used as a positive electrode material of a lithium secondary battery.

In particular, the sulfur-carbon composite may be used as a positive electrode material of a lithium-sulfur secondary battery, and even when sulfur contained in the sulfur coating layer is dissolved in the form of polysulfide according to the progress of charging/discharging of the battery, the surface energy of the electrolyte solution is lowered due to the fluorine-based surfactant coating layer, so that the wettability of the electrolyte solution to the positive electrode is excellent, and thus the chemical reactions occurring at the positive electrode can be uniformly generated.

In addition, when the sulfur-carbon composite is applied to a battery containing lithium metal as a negative electrode material, the deterioration of lithium can be prevented due to the fluorine-based surfactant coating layer, thereby improving the lifetime of the battery.

### Method for preparing sulfur-carbon composite

The present invention also relates to a method for preparing a sulfur-carbon composite comprising the steps of S1) dissolving the fluorine-based surfactant (fluorosurfactant) represented by Formula 1 in a solvent to prepare a fluorine-based surfactant solution; (S2) preparing a slurry by mixing the fluorine-based surfactant solution with a porous carbon material; (S3) drying the slurry to remove the solvent and form a slurry powder; (S4) mixing the slurry powder and sulfur to form a mixture; and (S5) heating the mixture at a heating temperature of 115 °C or more to prepare a sulfur-carbon composite.

Hereinafter, a method of preparing the sulfur-carbon composite of the present invention will be described in more detail at each step.

### Step (S1)

In step (S1), the fluorine-based surfactant is dissolved in a solvent to prepare a fluorine-based surfactant solution.

The solvent may be at least one selected from the group consisting of methanol, ethanol, 1-propanol, 2-butanol, isobutanol, 3-pentanol, dodecanol, chloroform, carbon tetrachloride, trichloroethylene, tetrachloroethylene, N-methyl-2-pyrrolidinone (NMP), and dimethyl formamide (DMF), and preferably may be ethanol.

The concentration of the fluorine-based surfactant solution may be 0.1 to 5 wt.%, preferably 0.1 to 2 wt.%, more preferably 0.1 to 1 wt.%, based on the weight of the solid components. If the concentration of the fluorine-based surfactant solution is less than the above range, the fluorine-based surfactant coating layer coated on the surface of the porous carbon material in the sulfur-carbon composite to be produced is not uniformly coated or the amount of fluorine-based surfactant may be small, so that the effects such as improvement of the wettability of the positive electrode due to the decrease of the surface energy of the electrolyte solution may not be expected. If the concentration exceeds the above range, since the concentration of the solution is excessively high, the excess surfactant acts as a resistance component to increase the overvoltage of the battery, and the fluorine-based surfactant coating layer may not be uniformly formed on the surface of the porous carbon material.

### Step (S2)

In step (S2), the fluorine-based surfactant solution and the porous carbon material may be mixed to prepare a slurry.

At this time, the amount of the used fluorine-based surfactant and the porous carbon material may be used as long as it satisfies the weight of the fluorine-based surfactant and the porous carbon material contained in the sulfur-carbon composite as described above.

In addition, the type and shape of the porous carbon material are as described above.

### Step (S3)

In step (S3), the slurry may be dried to form a slurry powder by removing the solvent.

The drying may be performed by stirring the slurry or heating it while stirring under vacuum.

At this time, if the solvent is removed by stirring the slurry under vacuum, the vacuum degree by the vacuum can be higher than the vapor pressure of the solvent to be used to limit the boiling of the solvent. In general, since the vacuum refers to an air pressure lower than the normal pressure, when assuming a vapor pressure of the solvent at a predetermined drying temperature, if a strong vacuum up to a pressure lower than the corresponding vapor pressure is applied to, the solvent is boiled, and thus uniform drying is not possible. Therefore, in order to dry without boiling the solvent by applying only the vacuum above the vapor pressure of the solvent, a vacuum above the vapor pressure of the solvent may be applied.

In addition, when removing the solvent by heating while stirring the slurry, the heating temperature may be below the boiling point of the solvent. For example, if ethanol was used as a solvent in the preparation of the fluorine-based surfactant solution, the drying may be performed by stirring and heating the slurry to a temperature of 78 °C or lower, which is the boiling point of ethanol, to remove the solvent.

After the solvent is removed, a slurry powder may be obtained, and the slurry powder may have a form in which a fluorine-based surfactant coating layer is formed on the surface of the porous carbon material.

### Step (S4)

In step (S4), the slurry powder and sulfur may be mixed to form a mixture.

In this case, the amount of the used sulfur may be used as long as it satisfies the weight of sulfur contained in the sulfur-carbon composite. In addition, the type and form of sulfur are as described above.

### Step (S5)

In step (S5), the mixture may be heated to prepare a sulfur-carbon composite.

At this time, the heating temperature is 115 °C or more, preferably 130°C to 180 °C, more preferably 150°C to 160 °C. If the heating temperature is less than the above range, sulfur is present in an unmelted state, and thus it may not be possible to form a sulfur coating layer. If the heating temperature exceeds the above range, sulfur-carbon composites can be prepared, but sulfur volatilization occurs, and thus loss of sulfur and deterioration of manufacturing equipment can be caused. In addition, since the viscosity of the molten sulfur is low at 150°C to 160 °C, it may be advantageous to support it.

Specifically, the mixture may be heated to prepare a composite by melt diffusion.

### Positive electrode

The present invention also relates to a positive electrode comprising a sulfur-carbon composite as described above.

The positive electrode may comprise a positive electrode current collector and a positive electrode active material layer formed on at least one surface of the positive electrode current collector. The positive electrode current collector is not particularly limited as long as it has high conductivity without causing chemical changes in the battery. For example, stainless steel, aluminum, nickel, titanium, sintered carbon; or aluminum or stainless steel surface-treated with carbon, nickel, titanium, silver or the like may be used as the positive electrode current collector. In this case, the positive electrode current collector may have various forms such as a film, a sheet, a foil, a net, a porous body, foam, and a nonwoven fabric having fine irregularities formed on a surface thereof so as to increase adhesion to the positive electrode active material.

In addition, the positive electrode active material layer may comprise a positive electrode active material, a binder, and an electrically conductive material.

In this case, the positive electrode active material may be the sulfur-carbon composite as described above.

The positive electrode active material may be contained in an amount of 40 to 95 wt.%, preferably 45 to 85 wt.%, more preferably 50 to 85 wt.%, based on the total weight of the positive electrode active material layer. If the content of the positive electrode active material is less than the above range, the capacity of the battery may be lowered. If the content of the positive electrode active material is larger than the above range, the content of the binder and the electrically conductive material may be relatively decreased, thereby decreasing the mechanical physical property and electrical conductivity of the positive electrode.

In addition, the binder is to increase the binding force between the positive electrode active material and the current collector, and may be poly(vinyl acetate), polyvinyl alcohol, polyethylene oxide, polyvinylpyrrolidone, alkylated polyethylene oxide, crosslinked polyethylene oxide, polyvinyl ether, poly(methyl methacrylate), polyvinylidene fluoride (PVDF), copolymers of polyhexafluoropropylene and polyvinylidene fluoride, poly(ethylacrylate), polytetrafluoroethylene, polyvinylchloride, polyacrylonitrile, polyvinylpyridine, polystyrene, styrenebutadiene rubber (SBR), carboxymethyl cellulose (CMC), and derivatives, blends, and copolymers thereof. In the case of the blend, a binder in the form of a mixture of the styrenebutadiene rubber and the carboxymethyl cellulose is exemplified.

In addition, the binder may be contained in an amount of 5 to 20 wt.%, preferably 5 to 18 wt.%, more preferably 5 to 15 wt.% based on the total weight of the positive electrode active material layer. If the content of the binder is less than the above range, the effect of improving the adhesion between the positive electrode active materials or between the positive electrode active material and the current collector depending on the use of the binder is insufficient. If the content of the binder exceeds the above range, the content of the positive electrode active material becomes relatively small, and thus there is a possibility that the capacity characteristics may be deteriorated.

The electrically conductive material is to allow electrons to move smoothly within the positive electrode, and may be a carbon-based material such as carbon black, acetylene black, and Ketjen black; or a conductive polymer such as polyaniline, polythiophene, polyacetylene, and polypyrrole.

In addition, the electrically conductive material may be contained in an amount of 5 to 40 wt.%, preferably 5 to 35 wt.%, more preferably 5 to 30 wt.% based on the total weight of the positive electrode active material layer. If the content of the electrically conductive material is less than the above range, the effect of improving conductivity due to the use of the electrically conductive material is insignificant. On the other hand, if the content of the electrically conductive material exceeds the above range, the content of the positive electrode active material may be relatively lowered, thereby deteriorating capacity characteristics.

### Method for preparing positive electrode

The positive electrode as described above may be prepared by a conventional method, and specifically, can be prepared by applying a composition for forming the positive electrode active material layer on a current collector, followed by drying and optionally rolling, wherein the composition is prepared by mixing the positive electrode active material, the conductive material, and the binder in an organic solvent,

In this case, the organic solvent may uniformly disperse the positive electrode active material, the binder, and the electrically conductive material, and is not particularly limited as long as it is easily evaporated. For example, the organic solvent may be at least one selected from the group consisting of acetonitrile, methanol, ethanol, tetrahydrofuran, water, isopropyl alcohol and the like.

### Lithium secondary battery

The present invention also relates to a lithium secondary battery in which the sulfur-carbon composite as described above is applied to a positive electrode.

The lithium secondary battery according to the present invention comprises a positive electrode, a negative electrode, and an electrolyte interposed therebetween, wherein a positive electrode containing the sulfur-carbon composite as described above as a positive electrode active material may be used as the positive electrode.

In addition, in the lithium secondary battery according to the present invention, the negative electrode is manufactured by forming a negative electrode active material layer with a negative electrode active material on the negative electrode current collector, or may be a negative electrode active material layer (for example, lithium foil) alone.

At this time, the types of the negative electrode current collector and the negative electrode active material layer are not particularly limited in the present invention, and known materials can be used.

In addition, the negative electrode current collector is not particularly limited as long as it has electrical conductivity without causing a chemical change in the battery. For example, copper, stainless steel, aluminum, nickel, titanium, sintered carbon, copper, or stainless steel surface-treated with carbon, nickel, titanium, silver or the like; aluminum-cadmium alloy or the like may be used as the negative electrode current collector. Also, as with the positive electrode current collector, the shape of the negative electrode current collector can be various forms such as a film having fine irregularities on its surface, a sheet, a foil, a net, a porous body, foam, a nonwoven fabric and the like.

In addition, the negative electrode active material may comprises, but is not limited to, at least one carbon-based material selected from the group consisting of crystalline artificial graphite, crystalline natural graphite, amorphous hard carbon, low crystalline soft carbon, carbon black, acetylene black, Ketjen black, Super-P, graphene, and fibrous carbon, Si-based material, metal composite oxides such as LixFe₂O₃(0≤x≤1), LiₓWO₂(0≤x≤1), SnₓMe₁₋ₓMe'_{y}O_{z} (Me: Mn, Fe, Pb, Ge; Me': Al, B, P, Si, elements of groups 1, 2, and 3 of the periodic table, halogen; 0<x≤1; 1≤y≤3; 1≤z≤8); lithium metal; lithium alloy; silicon-based alloy; tin-based alloy; metal oxide such as SnO, SnO₂, PbO, PbO₂, Pb₂O₃, Pb₃O₄, Sb₂O₃, Sb₂O₄, Sb₂O₅, GeO, GeO₂, Bi₂O₃, Bi₂O₄, Bi₂O₅; an electrically conductive polymer such as polyacetylene; Li-Co-Ni based material; titanium oxide; lithium titanium oxide.

In addition, the negative electrode active material may be metal composite oxides such as SnxMe₁₋ₓMe'_{y}O_{z} (Me: Mn, Fe, Pb, Ge; Me': Al, B, P, Si, elements of groups 1, 2, and 3 of the periodic table, halogen; 0<x≤1; 1≤y≤3; 1≤z≤8); oxides such as SnO, SnO₂, PbO, PbO₂, Pb₂O₃, Pb₃O₄, Sb₂O₃, Sb₂O₄, Sb₂O₅, GeO, GeO₂, Bi₂O₃, Bi₂O₄, and Bi₂O₅, and may be carbon-based negative electrode active materials such as crystalline carbon, amorphous carbon, or carbon composite alone or in combination of two or more.

In addition, in the lithium secondary battery according to the present invention, any of those conventionally used in the manufacture of a lithium secondary battery can be used as an electrolyte solution.

For example, lithium salts that may be included as electrolytes in the electrolyte solution may be used without limitation as long as they are commonly used in electrolyte solutions for a lithium secondary battery. For example, the anion of the lithium salt may be any one selected from the group consisting of F⁻, Cl⁻, Br⁻, I⁻, NO₃⁻, N(CN)₂⁻, BF₄⁻, ClO₄⁻, PF₆⁻, (CF₃)₂PF₄⁻, (CF₃)₃PF₃⁻, (CF₃)₄PF₂⁻, (CF₃)₅PF⁻, (CF₃)₆P⁻, CF₃SO₃⁻, CF₃CF₂SO₃⁻, (CF₃SO₂)₂N⁻, (FSO₂)₂N⁻, CF₃CF₂(CF₃)₂CO⁻, (CF₃SO₂)₂CH⁻, (SF₅)₃C⁻, (CF₃SO₂)₃C⁻, CF₃(CF₂)₇SO₃⁻, CF₃CO₂⁻, CH₃CO₂⁻, SCN⁻, and (CF₃CF₂SO₂)₂N⁻.

In the electrolyte solution used in the present invention, the organic solvent contained in the electrolyte solution may be used without limitation so long as they are conventionally used in the electrolyte solution for a lithium secondary battery. Typically, at least one selected from the group consisting of propylene carbonate (PC), ethylene carbonate (EC), diethyl carbonate (DEC), dimethyl carbonate (DMC), ethylmethyl carbonate (EMC), methylpropyl carbonate, dipropyl carbonate, dimethylsulfuroxide, acetonitrile, dimethoxyethane, diethoxyethane, vinylene carbonate, sulfolane, gamma-butyrolactone, propylene sulfite, and tetrahydrofuran, and a mixture of two or more of them, and the like can be used representatively. In particular, ethylene carbonate and propylene carbonate which are cyclic carbonates among the carbonate-based organic solvents are highly viscous organic solvents, which can be preferably used because they have a high dielectric constant and dissociate lithium salts in the electrolyte well. If such cyclic carbonates are mixed with a linear carbonate having a low viscosity and a low dielectric constant, such as dimethyl carbonate and diethyl carbonate in an appropriate ratio, an electrolyte solution having a high electrical conductivity can be made, and thus it can be used more preferably.

In addition, in the lithium secondary battery according to the present invention, the separator may be a conventional porous polymer film conventionally used as a separator. For example, as the separator, a porous polymer film made of a polyolefin-based polymer such as ethylene homopolymer, propylene homopolymer, ethylene/butene copolymer, ethylene/hexene copolymer, and ethylene/methacrylate copolymer may be used alone or they may be laminated and used, or a conventional porous nonwoven fabric, for example, a non-woven fabric made of high melting glass fibers, polyethylene terephthalate fibers, or the like may be used, but is not limited thereto.

The type of lithium secondary battery as described above is not particularly limited, and may be, for example, a jelly-roll type, a stack type, a stack-folding type (including a stack-Z-folding type), or a lamination-stack type, preferably a stack-folding type.

The negative electrode, the separator, and the positive electrode as described above are sequentially stacked, and the electrolyte solution is injecting to prepare an electrode assembly, and then the electrode assembly is placed in a battery case and sealed with a cap plate and a gasket to manufacture a lithium secondary battery.

In this case, the lithium secondary battery can be classified into various types of batteries such as lithium-sulfur secondary battery, lithium-air battery, lithium-oxide battery, and lithium all-solid-state battery depending on the materials of positive electrode/negative electrode used, can be classified into cylindrical, rectangular, coin-shaped, pouch type depending on the type, and can be divided into bulk type and thin film type depending on the size. The structure and preparation method of these batteries are well known in the art, and thus detailed description thereof is omitted.

In the lithium-sulfur secondary battery, sulfur may be used as a positive electrode active material, and lithium metal may be used as a negative electrode active material. When the lithium-sulfur secondary battery is discharged, the oxidation of lithium occurs on the negative electrode and the reduction of sulfur occurs on the positive electrode. In this case, the reduced sulfur combines with lithium ions that have been transported from the negative electrode, and thus is converted to lithium polysulfide, and then finally is accompanied by a reaction that forms lithium sulfide.

The lithium secondary battery according to the present invention can be used as a power source for devices requiring high capacity and high rate characteristics, etc. Specific examples of the device may comprise, but are not limited to, a power tool that is powered by a battery powered motor; electric cars including an electric vehicle (EV), a hybrid electric vehicle (HEV), a plug-in hybrid electric vehicle (PHEV), and the like; an electric motorcycle including an electric bike (E-bike) and an electric scooter (Escooter); an electric golf cart; and a power storage system.

### Example 1

### (1) Preparation of sulfur-carbon composite

A fluorine-based surfactant (FC-4430, 3M^{™}, CAS No.: 1017237-78-3) was dissolved in ethanol solvent to prepare a 0.5 wt.% fluorine-based surfactant solution based on the weight of the solid components.

The fluorine-based surfactant solution and the porous carbon material (carbon powder, CAS No.: 308068-56-6) were uniformly mixed to prepare a slurry.

The slurry was heated to 70 °C while stirring to remove the solvent, thereby obtaining a slurry powder.

The mixture obtained by mixing the slurry powder and sulfur powder (CAS No.: 7704-34-9) was heated at 155 °C to prepare a sulfur-carbon composite by melt diffusion method.

In this case, in the prepared sulfur-carbon composite, the weight of carbon, fluorine-based surfactant, and sulfur is 28.5 wt.%, 1.5 wt.%, and 70.0 wt.%, respectively.

### (2) Preparation of positive electrode

Foamed aluminum was prepared as a positive electrode current collector.

75 wt.% of the sulfur-carbon composite, 10 wt.% of the electrically conductive material, and 15 wt.% of the binder are mixed and dissolved in deionized water (DI water) as a solvent to prepare a slurry having a concentration of 5 wt.% based on the solid components. Thereafter, the obtained slurry was applied and dried on the positive electrode current collector to prepare a positive electrode. At this time, the electrically conductive material was carbon black, and the binder was PVDF binder.

### (3) Preparation of lithium-sulfur secondary battery

After placing the positive electrode containing the prepared sulfur-carbon composite and the lithium negative electrode to face each other, the polyethylene separator having a thickness of 20 um and a porosity of 45% was interposed between the positive electrode and the negative electrode.

Thereafter, the electrolyte solution was injected into the case to prepare a lithium-sulfur secondary battery. At this time, the electrolyte was prepared by dissolving lithium bis(fluorosulfonyl)imide (LiFSI) of a concentration of 1M and 1 wt.% of LiNO₃ in an organic solvent consisting of dioxolane (DOL) and dimethylene glycol dimethyl ether (DEGDME) (mixing volume ratio = 6 : 4).

### Example 2 (not according to the invention)

A sulfur-carbon composite, a positive electrode, and a lithium secondary battery were prepared in the same manner as in Example 1, except that in the prepared sulfur-carbon composite, the weight of carbon, fluorine-based surfactant, and sulfur was 29.95 wt.%, 0.05 wt.%, and 70.0 wt.%, respectively.

### Example 3 (not according to the invention)

A sulfur-carbon composite, a positive electrode, and a lithium secondary battery were prepared in the same manner as in Example 1, except that in the prepared sulfur-carbon composite, the weight of carbon, fluorine-based surfactant, and sulfur was 26 wt.%, 4 wt.%, and 70.0 wt.%, respectively.

### Comparative Example 1

A sulfur-carbon composite, a positive electrode, and a lithium secondary battery were prepared in the same manner as in Example 1, except that the fluorine-based surfactant was not used and the weight ratio of sulfur and carbon was 3 : 7.

### Comparative Example 2

A sulfur-carbon composite was prepared in the same manner as in Example 1 and applied to the positive electrode, except that the fluorine-based surfactant was not used and the weight ratio of sulfur and carbon was 3 : 7.

In addition, a lithium-sulfur secondary battery was prepared by injecting 0.3 wt.% of the fluorine-based surfactant into the electrolyte solution.

### Comparative Example 3

30 wt.% of carbon particles and 70 wt.% of sulfur were mixed to prepare a composite by melt diffusion method. Thereafter, the positive electrode was prepared in the same manner by adding fluorine-based surfactant in an amount of 1 wt.% based on the weight of the composite, when adding D.I water in the preparation process of the positive electrode. A lithium-sulfur secondary battery was prepared in the same manner as in Example 1.

### Experimental Example 1

Experiments were conducted on the capacity and lifetime characteristics of the batteries prepared in the examples and the comparative examples.

For the batteries manufactured in the examples and the comparative examples, after discharging to 0.1C, charging/discharging was performed twice, and 0.1C charging/0.2C discharging was performed three times. Thereafter, the operation condition was set to 0.3C charging/0.5C discharging.

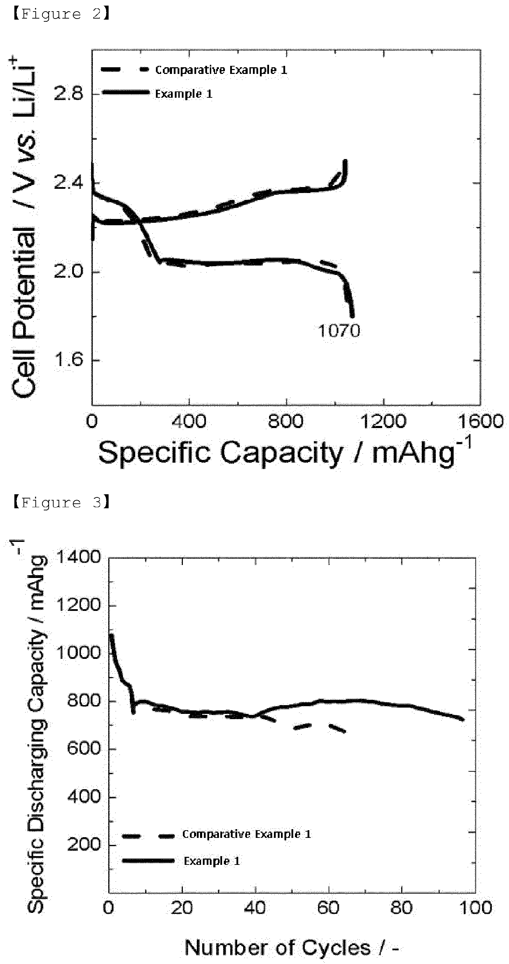

FIG. 2 is a graph showing the discharging capacity measured for the lithium-sulfur secondary batteries prepared in Example 1 of the present invention and Comparative Example 1, and FIG. 3 is a graph showing the lifetime characteristics measured for the lithium-sulfur secondary batteries prepared in Example 1 of the present invention and Comparative Example 1.

Referring to FIGs. 2 and 3, it can be seen that the discharging capacity per weight of sulfur was the same, but the lifetime of Example 1 was improved by about 2 times compared to Comparative Example 1.

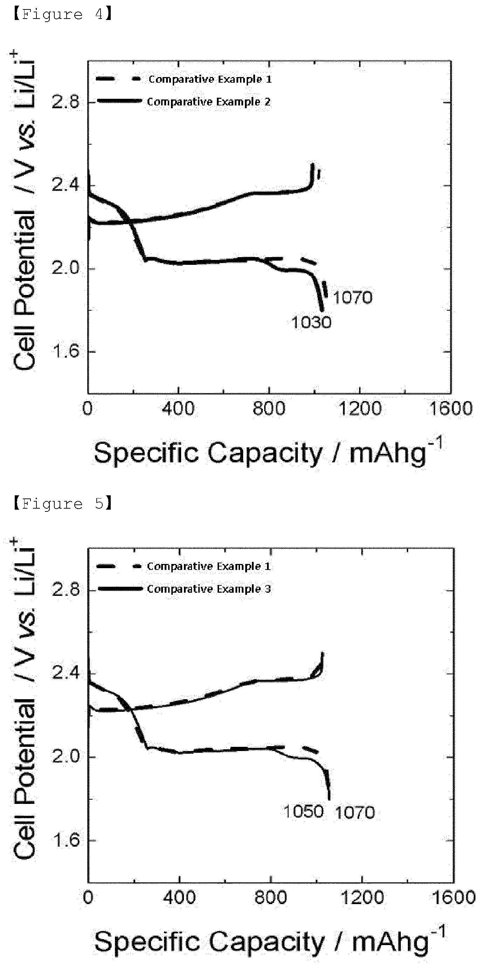

FIG. 4 is a graph showing the discharging capacity measured for the lithium-sulfur secondary batteries prepared in Comparative Example 1 and Comparative Example 2 of the present disclosure.

Referring to FIG. 4, it can be seen that Comparative Example 2 has a lower discharge capacity than Comparative Example 1. From this, it can be confirmed that the performance of the battery is further reduced in the case where the fluorine-based surfactant is contained in the electrolyte solution rather than the positive electrode, as compared to the case where the fluorine-based surfactant is not contained in both the positive electrode and the electrolyte solution.

FIG. 5 is a graph showing the discharging capacity measured for the lithium-sulfur secondary batteries prepared in Comparative Example 1 and Comparative Example 3 of the present disclosure.

Referring to FIG. 5, it can be seen that Comparative Example 3 shows a lower discharging capacity compared to Comparative Example 1. From this, it can be confirmed that the performance of the battery is further reduced in the case where the fluorine-based surfactant is included in the slurry for the preparation of the positive electrode, as compared to the case where the fluorine-based surfactant is coated between the carbon material and sulfur.

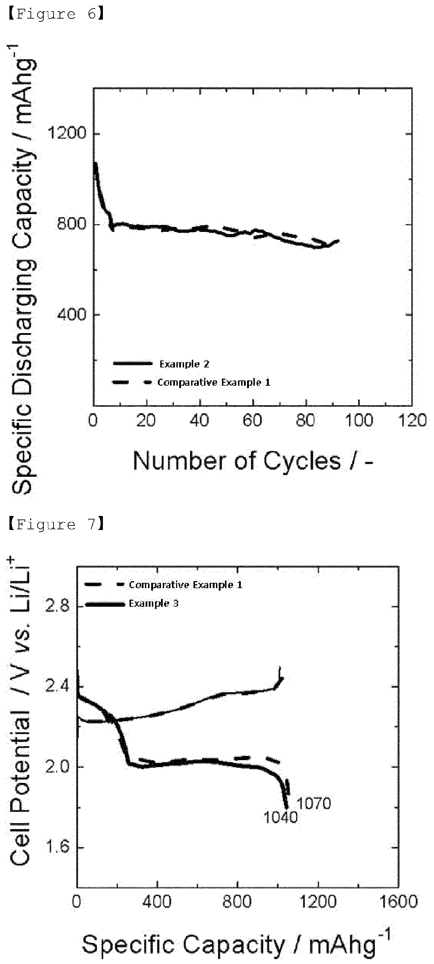

FIG. 6 is a graph showing the lifetime characteristics measured for the lithium-sulfur secondary batteries prepared in Example 2 and Comparative Example 1 of the present disclosure.

Referring to FIG. 6, it can be seen that Example 2 contains a little smaller fluorine-based surfactant than that of Example 1 and has a slight improvement in lifetime characteristics compared to Comparative Example 1 which does not contain fluorine-based surfactant.

FIG. 7 is a graph showing the discharging capacity measured for the lithium-sulfur secondary batteries prepared in Example 3 of the present disclosure and Comparative Example 1.

Referring to FIG. 7, it can be seen that Example 3 contains somewhat more fluorine-based surfactant than that of Example 1 and has a slight improvement in the discharging capacity compared to Comparative Example 1, which does not contain fluorine-based surfactant. In addition, it was confirmed that Example 3 has an increased discharging terminal resistance and a reduced discharging capacity.

### Experimental Example 2

The shape of the cross section of the sulfur-carbon composites prepared in the examples and the comparative examples was observed.

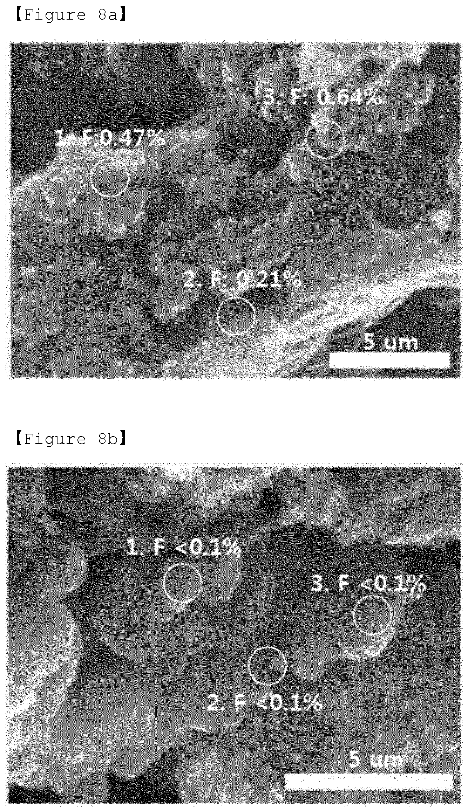

FIGs. 8a and 8b are Scanning Electron Microscope (SEM) photographs of the cross section of the sulfur-carbon composite prepared in Example 1.

Referring to FIG. 8a, it can be seen that after coating the carbon material of the example with a fluorine-based surfactant, a small amount of F atoms are observed on the surface, but since F atoms are not observed after supporting sulfur thereon, as shown in FIG. 8b, the fluorine-based surfactant in the sulfur-carbon composite of Example 1 exists between the carbon material and sulfur. The numerals described in FIGs. 8a and 8b all represent the fraction of F.

## Claims

1. A sulfur-carbon composite comprising
a porous carbon material;
a fluorine-based surfactant coating layer formed on the surface of the porous carbon material and an inner surface of pores of the porous carbon material; and
a sulfur coating layer formed on the surface of the fluorine-based surfactant coating layer opposite the porous carbon material,
wherein the sulfur-carbon composite contains 9 wt.% to 49 wt.% of the porous carbon material; 0.1 wt.% to 3 wt.% of fluorine-based surfactant; and 50 wt.% to 90 wt.% of sulfur; and
wherein the fluorine-based surfactant is represented by Formula 1 below: 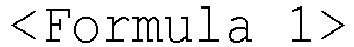 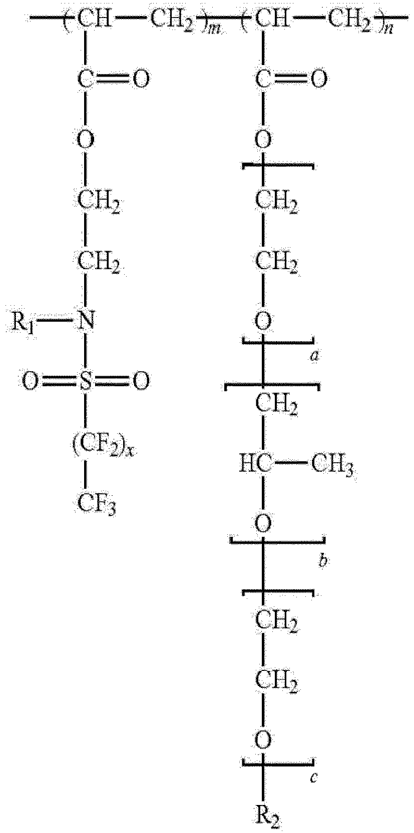
wherein m and n are each an integer and m/(m + n) is a real number from 0 to 1;
R₁ is hydrogen or an alkyl group having 1 to 18 carbon atoms;
R₂ is a connection point for hydrogen, an alkyl group having 1 to 18 carbon atoms or an acrylate polymer backbone;
x is an integer from 1 to 10;
a is an integer from 1 to 50;
b is an integer from 1 to 100; and
c is an integer from 1 to 50.

2. The sulfur-carbon composite according to claim 1, wherein the porous carbon material comprises at least one selected from the group consisting of graphite, graphene, carbon black, carbon nanotubes, carbon fibers, and activated carbon.

3. The sulfur-carbon composite according to claim 1, wherein the sulfur comprises at least one selected from the group consisting of inorganic sulfur (S₈), Li₂Sₙ wherein n ≥ 1 and n is an integer, organic sulfur compound, and carbon-sulfur polymer of formula (C₂Sₓ)ₙ, wherein 2.5 ≤ x ≤ 50, n ≥ 2, and x and n are integers.

4. A method for preparing a sulfur-carbon composite comprising the steps of:
(S1) dissolving a fluorine-based surfactant in a solvent to prepare a fluorine-based surfactant solution;
(S2) preparing a slurry by mixing the fluorine-based surfactant solution with a porous carbon material;
(S3) drying the slurry to remove the solvent and form a slurry powder;
(S4) mixing the slurry powder and sulfur to form a mixture; and
(S5) heating the mixture at a heating temperature of 115 °C or more to prepare a sulfur-carbon composite;
wherein the fluorine-based surfactant is represented by Formula 1 below: 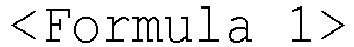 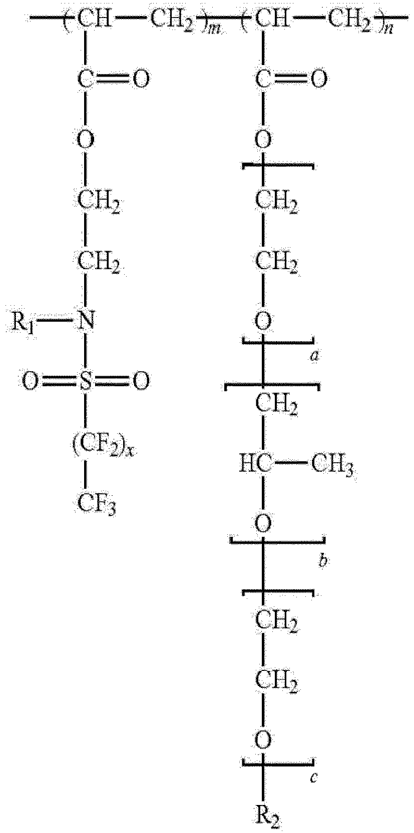
wherein m and n are each an integer and m/(m + n) is a real number from 0 to 1;
R₁ is hydrogen or an alkyl group having 1 to 18 carbon atoms;
R₂ is a connection point for hydrogen, an alkyl group having 1 to 18 carbon atoms or an acrylate polymer backbone;
x is an integer from 1 to 10;
a is an integer from 1 to 50;
b is an integer from 1 to 100; and
c is an integer from 1 to 50.

5. The method for preparing a sulfur-carbon composite according to claim 4, wherein in step (S1), the solvent comprises at least one selected from the group consisting of methanol, ethanol, 1-propanol, 2-butanol, isobutanol, 3-pentanol, dodecanol, chloroform, carbon tetrachloride, trichloroethylene, tetrachloroethylene, N-methyl-2-pyrrolidinone (NMP), and dimethyl formamide (DMF).

6. The method for preparing a sulfur-carbon composite according to claim 4, wherein a concentration of the fluorine-based surfactant solution is 0.1 wt.% to 10 wt.% based on a total weight of solid components.

7. The method for preparing a sulfur-carbon composite according to claim 4, wherein in step (S3), the drying may be performed by stirring the slurry or heating the slurry while stirring under vacuum.

8. A positive electrode for a lithium secondary battery comprising the sulfur-carbon composite of any one of claims 1 to 3.

9. A lithium secondary battery comprising the positive electrode of claim 8.

10. The lithium secondary battery according to claim 9, wherein the lithium secondary battery is a lithium-sulfur secondary battery.

## Patentansprüche

1. Schwefel-Kohlenstoff-Komposit, umfassend
ein poröses Kohlenstoffmaterial;
eine fluorbasierte Tensid-Beschichtungsschicht, gebildet auf der Oberfläche des porösen Kohlenstoffmaterials und einer inneren Oberfläche von Poren des porösen Kohlenstoffmaterials; und
eine Schwefel-Beschichtungsschicht, gebildet auf der dem porösen Kohlenstoffmaterial gegenüberliegenden Oberfläche der fluorbasierten Tensid-Beschichtungsschicht,
worin das Schwefel-Kohlenstoff-Komposit 9 Gew.-% bis 49 Gew.-% des porösen Kohlenstoffmaterials; 0,1 Gew.-% bis 3 Gew.-% des fluorbasierten Tensids; und 50 Gew.-% bis 90 Gew.-% Schwefel enthält; und
worin das fluorbasierte Tensid durch die nachstehende Formel 1 dargestellt wird: 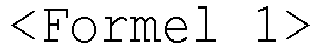 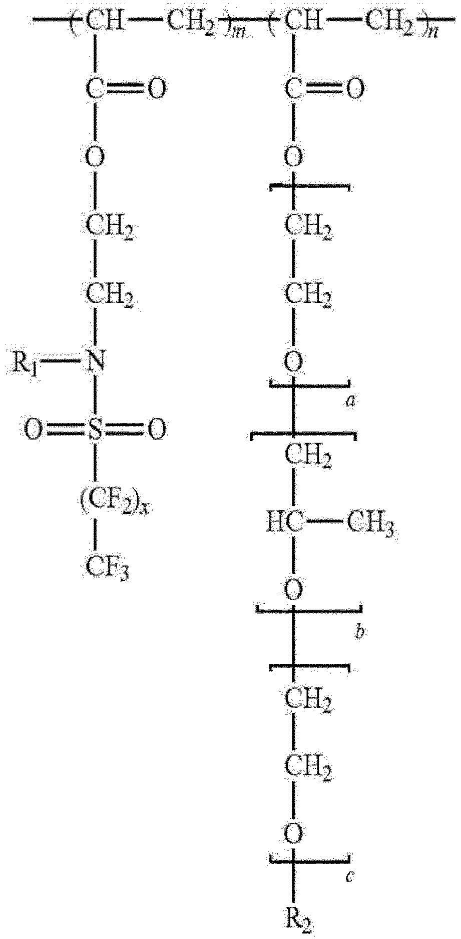
worin m und n jeweils eine ganze Zahl sind und m/(m + n) eine reale Zahl von 0 bis 1 ist;
R₁ Wasserstoff oder eine Alkylgruppe mit 1 bis 18 Kohlenstoffatomen ist;
R₂ ein Verbindungspunkt für Wasserstoff, eine Alkylgruppe mit 1 bis 18 Kohlenstoffatomen oder ein Acrylat-Polymergerüst ist;
x eine ganze Zahl von 1 bis 10 ist;
a eine ganze Zahl von 1 bis 50 ist;
b eine ganze Zahl von 1 bis 100 ist; und
c eine ganze Zahl von 1 bis 50 ist.

2. Schwefel-Kohlenstoff-Komposit gemäß Anspruch 1, worin das poröse Kohlenstoffmaterial mindestens eines umfasst, das ausgewählt ist aus der Gruppe bestehend aus Graphit, Graphen, Ruß, Kohlenstoff-Nanoröhrchen, Kohlefasern und Aktivkohle.

3. Schwefel-Kohlenstoff-Komposit gemäß Anspruch 1, worin der Schwefel zumindest eines umfasst, das ausgewählt ist aus der Gruppe bestehend aus anorganischem Schwefel (S₈), Li₂Sₙ, worin n ≥ 1 ist und n eine ganze Zahl ist, organischen Schwefelverbindungen und Kohlenstoff-Schwefel-Polymeren der Formel (C₂Sₓ)ₙ, worin 2,5 ≤ x ≤ 50, n ≥ 2 und x und n ganze Zahlen sind.

4. Verfahren zur Herstellung eines Schwefel-Kohlenstoff-Komposits, umfassend die Schritte:
(S1) Auflösen eines fluorbasierten Tensids in einem Lösungsmittel, um eine fluorbasierte Tensidlösung herzustellen,
(S2) Zubereiten einer Aufschlämmung durch Mischen der fluorbasierten Tensidlösung mit einem porösen Kohlenstoffmaterial;
(S3) Trocknen der Aufschlämmung, um fas Lösungsmittel zu entfernen und ein Aufschlämmungspulver zu bilden;
(S4) Mischen des Aufschlämmungspulvers und Schwefel, um eine Mischung zu bilden; und
(S5) Erwärmen der Mischung auf eine Erwärmungstemperatur von 115°C oder höher, um ein Schwefel-Kohlenstoff-Komposit herzustellen;
worin das fluorbasierte Tensid durch die nachstehende Formel 1 dargestellt wird: 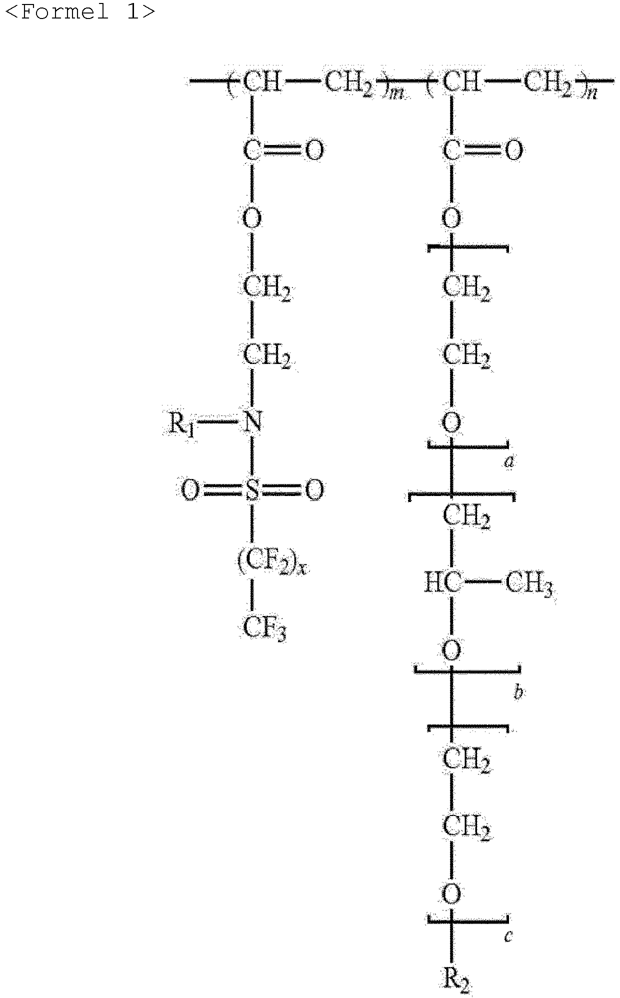
worin m und n jeweils eine ganze Zahl sind und m/(m + n) eine reale Zahl von 0 bis 1 ist;
R₁ Wasserstoff oder eine Alkylgruppe mit 1 bis 18 Kohlenstoffatomen ist;
R₂ ein Verbindungspunkt für Wasserstoff, eine Alkylgruppe mit 1 bis 18 Kohlenstoffatomen oder ein Acrylat-Polymergerüst ist;
x eine ganze Zahl von 1 bis 10 ist;
a eine ganze Zahl von 1 bis 50 ist;
b eine ganze Zahl von 1 bis 100 ist; und
c eine ganze Zahl von 1 bis 50 ist.

5. Verfahren zur Herstellung eines Schwefel-Kohlenstoff-Komposits gemäß Anspruch 4, worin in dem Schritt (S1) das Lösungsmittel zumindest eines umfasst, das ausgewählt ist aus der Gruppe bestehend aus Methanol, Ethanol, 1-Propanol, 2-Butanol, Isobutanol, 3-Pentanol, Dodecanol, Chloroform, Tetrachlorkohlenstoff, Trichlorethylen, Tetrachlorethylen, N-Methyl-2-pyrrolidinon (NMP) und Dimethylformamid (DMF).

6. Verfahren zur Herstellung eines Schwefel-Kohlenstoff-Komposits gemäß Anspruch 4, worin die Konzentration der fluorbasierten Tensidlösung 0,1 Gew.-% bis 10 Gew.-% beträgt, bezogen auf das Gesamtgewicht der festen Komponenten.

7. Verfahren zur Herstellung eines Schwefel-Kohlenstoff-Komposits gemäß Anspruch 4, worin im Schritt (S3) das Trocknen durchgeführt werden kann, indem die Aufschlämmung gerührt wird oder die Aufschlämmung unter Rühren im Vakuum erwärmt wird.

8. Positive Elektrode für eine Lithium-Sekundärbatterie, umfassend das Schwefel-Kohlenstoff-Komposit gemäß irgendeinem der Ansprüche 1 bis 3.

9. Lithium-Sekundärbatterie, umfassend die positive Elektrode gemäß Anspruch 8.

10. Lithium-Sekundärbatterie gemäß Anspruch 9, worin die Lithiumsekundärbatterie eine Lithium-Schwefel-Sekundärbatterie ist.

## Revendications

1. Composite soufre-carbone comprenant
un matériau carboné poreux ;
une couche de revêtement de tensioactif à base de fluor formée sur la surface du matériau carboné poreux et une surface intérieure de pores du matériau carboné poreux ; et
une couche de revêtement de soufre formée sur la surface de la couche de revêtement de tensioactif à base de fluor opposée au matériau carboné poreux,
dans lequel le composite soufre-carbone contient de 9 % en poids à 49 % en poids du matériau carboné poreux ; de 0,1 % en poids à 3 % en poids de tensioactif à base de fluor ; et de 50 % en poids à 90 % en poids de soufre ; et
dans lequel le tensioactif à base de fluor est représenté par la Formule 1 ci-dessous : 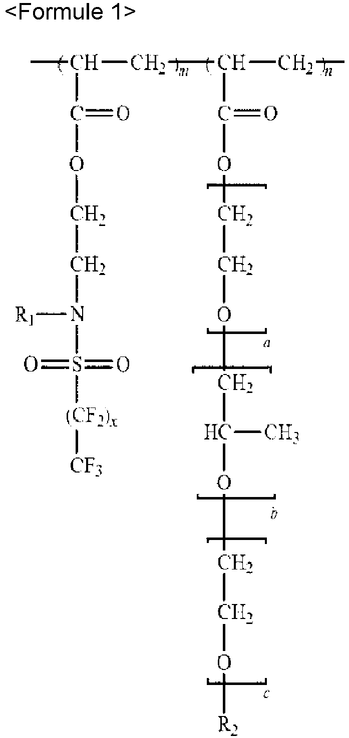
dans laquelle m et n sont chacun un nombre entier et m/(m + n) est un nombre réel de 0 à 1 ;
R₁ est l'hydrogène ou un groupe alkyle ayant de 1 à 18 atomes de carbone ;
R₂ est un point de liaison pour l'hydrogène, un groupe alkyle ayant de 1 à 18 atomes de carbone ou une chaîne principale de polymère acrylate ;
x est un nombre entier de 1 à 10 ;
a est un nombre entier de 1 à 50 ;
b est un nombre entier de 1 à 100 ; et
c est un nombre entier de 1 à 50.

2. Composite soufre-carbone selon la revendication 1, dans lequel le matériau carboné poreux comprend au moins un sélectionné à partir du groupe consistant en graphite, graphène, noir de carbone, nanotubes de carbone, fibres de carbone et charbon actif.

3. Composite soufre-carbone selon la revendication 1, dans lequel le soufre comprend au moins un sélectionné à partir du groupe consistant en soufre inorganique (S₈), Li₂Sₙ dans lequel n ≥ 1 et n est un nombre entier, composé de soufre organique et polymère carbone-soufre de formule (C₂Sₓ)ₙ, dans lequel 2,5 ≤ x ≤ 50, n ≥ 2, et x et n sont des nombres entiers.

4. Procédé de préparation d'un composite soufre-carbone comprenant les étapes de :
(S1) dissolution d'un tensioactif à base de fluor dans un solvant pour préparer une solution de tensioactif à base de fluor ;
(S2) préparation d'une suspension en mélangeant la solution de tensioactif à base de fluor avec un matériau carboné poreux ;
(S3) séchage de la suspension afin d'éliminer le solvant et de former une poudre de suspension ;
(S4) mélange de la poudre de suspension et du soufre afin de former un mélange ; et
(S5) chauffage du mélange à une température de chauffage de 115 °C ou plus pour préparer un composite soufre-carbone ;
dans lequel le tensioactif à base de fluor est représenté par la Formule 1 ci-dessous : 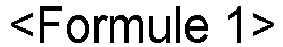 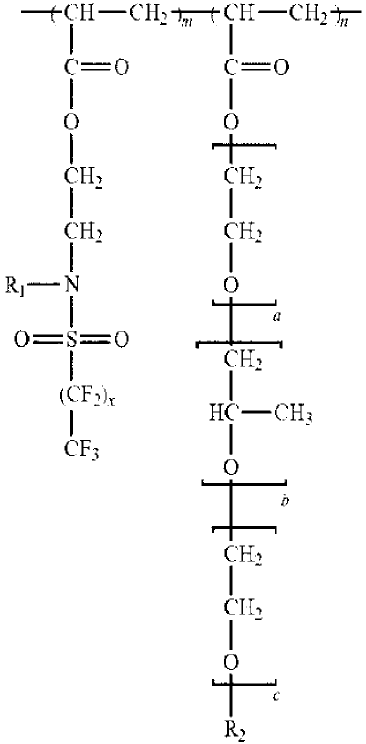
dans laquelle m et n sont chacun un nombre entier et m/(m + n) est un nombre réel de 0 à 1 ;
R₁ est l'hydrogène ou un groupe alkyle ayant de 1 à 18 atomes de carbone ;
R₂ est un point de liaison pour l'hydrogène, un groupe alkyle ayant de 1 à 18 atomes de carbone ou une chaîne principale de polymère acrylate ;
x est un nombre entier de 1 à 10 ;
a est un nombre entier de 1 à 50 ;
b est un nombre entier de 1 à 100 ; et
c est un nombre entier de 1 à 50.

5. Procédé de préparation d'un composite soufre-carbone selon la revendication 4, dans lequel à l'étape (S1), le solvant comprend au moins un sélectionné à partir du groupe consistant en méthanol, éthanol, 1-propanol, 2-butanol, isobutanol, 3-pentanol, dodécanol, chloroforme, tétrachlorure de carbone, trichloroéthylène, tétrachloroéthylène, N-méthyl-2-pyrrolidinone (NMP) et formamide de diméthyle (DMF).

6. Procédé de préparation d'un composite soufre-carbone selon la revendication 4, dans lequel une concentration de la solution de tensioactif à base de fluor est de 0,1 % en poids à 10 % en poids sur la base d'un poids total de composants solides.

7. Procédé de préparation d'un composite soufre-carbone selon la revendication 4, dans lequel à l'étape (S3), le séchage peut être mis en oeuvre en remuant la suspension ou en chauffant la suspension tout en la remuant sous vide.

8. Électrode positive pour un accumulateur au lithium comprenant le composite soufre-carbone selon l'une quelconque des revendications 1 à 3.

9. Accumulateur au lithium comprenant l'électrode positive selon la revendication 8.

10. Accumulateur au lithium selon la revendication 9, dans lequel l'accumulateur au lithium est un accumulateur au lithium-soufre.
